# EUROPÄISCHE PATENTSCHRIFT

(11) **EP 2 309 983 B1**
(45) Veröffentlichungstag und Bekanntmachung des Hinweises auf die Patenterteilung: **24.04.2019**
(21) Anmeldenummer: 09775591.2
(22) Anmeldetag: 07.08.2009
(51) Int. Cl.: A61K 9/08, A61K 31/519, A61P 3/00

(54) **PHARMAZEUTISCHE DARREICHUNGSFORM ENTHALTEND TETRAHYDROBIOPTERIN**
PHARMACEUTICAL FORM COMPRISING TETRAHYDROBIOPTERIN
FORME GALÉNIQUE PHARMACEUTIQUE CONTENANT DE LA TÉTRAHYDROBIOPTÉRINE

(30) Priorität: 12.08.2008 AT 12542008
(43) Veröffentlichungstag der Anmeldung: 20.04.2011
(73) Patentinhaber: Orpha Swiss GmbH, 8700 Küsnacht (CH)
(72) Erfinder: WIDMANN, Rudolf, A-3002 Purkersdorf (AT); BERNKOP-SCHNUERCH, Andreas, A-9300 St. Veit/Glan (AT)
(74) Vertreter: Nemec, Harald
(86) Internationale Anmeldenummer: PCT/AT2009/000306
(87) Internationale Veröffentlichungsnummer: WO 2010/017570

(56) Entgegenhaltungen:
- EP-A2- 0 209 689
- WO-A2-2004/058268
- WO-A2-2006/120176
- WO-A2-2008/128049
- US-A1- 2006 188 492
- DATABASE CA [Online] CHEMICAL ABSTRACTS SERVICE, COLUMBUS, OHIO, US; 28. Oktober 1989 (1989-10-28), XP002585278 gefunden im STN Database accession no. 111:146824 & JP 63 267781 A (SUNTORY LTD) 4. November 1988 (1988-11-04)
- S. VALENT ET AL.: "Spectrophotometric analysis of the protective effect of ascorbate against spontaneous oxidation of tetrahydrobiopterin in aqueous solution: kinetic characteristics and potentiation by catalase of ascorbate action" THE INTERNATIONAL JOURNAL OF BIOCHEMISTRY AND CELL BIOLOGY, Bd. 36, 2004, Seiten 1266-1280, XP002585279

## Beschreibung

Die vorliegende Erfindung betrifft eine pharmazeutische Darreichungsform enthaltend Tetrahydrobiopterin, die zur Behandlung von Phenylketonurie-Varianten, die durch einen Tetrahydrobiopterin-Mangel verursacht werden, Verwendung finden kann.

Tetrahydrobiopterin stellt einen Cofaktor der Phenylalaninhydroxylase (PAH) dar und ist auch ein Cofaktor der Alkylglycerolmonooxygenase, der Tyrosinhydroxylase (TH), der Tryptophanhydroxylase (TPH) und der Stickoxidhydroxylasen, bei denen Tetrahydrobiopterin zum ersten Mal identifiziert wurde. Daher ist Tetrahydrobiopterin für die Umwandlung von Phenylalanin in Tyrosin und für die Produktion der Monoamin-Neurotransmitter Dopamin, Epinephrin, Norepinephrin und Serotonin erforderlich (Miwa et al., Arch. Biochem. Biophys. 1985; 239: 234-241).

Hyperphenylalaninämie ist eine weitverbreitete angeborene Stoffwechselkrankheit, die in den meisten Fällen (ungefähr 98%) aus einem Phenylalaninhydroxylase-Mangel aufgrund von Mutationen im Phenylalaninhydroxylase-Gen entsteht (Scriver et al. Hum Mutat 2000; 15: 99-104). Die dazugehörigen Phänotypen reichen je nach Schwere von einer klassischen Phenylketonurie (> 1200 µmol/l Phenylalanin im Blutplasma) bis zu einer leichten Phenylketonurie (600-1200 µmol/l Phenylalanin im Blutplasma) und einer leichten Hyperphenylalaninämie (120-600 µmol/l Phenylalanin im Blutplasma). Patienten mit sowohl klassischer als auch schwacher Phenylketonurie benötigen eine lebenslange Diät mit Proteinrestriktion, um neurologische *Sequelae* zu verhindern und eine normale kognitive Entwicklung sicherzustellen.

Untersuchungen zeigten, dass eine Ernährungsergänzung mit Tetrahydrobiopterin die Phenylalanin-Blutplasmakonzentration bei Patienten, die Mutationen im PAH-Gen aufweisen, verringern kann (Kure et al. J Pediatr 1999; 135: 375-8), wobei aber offenbar nur bestimmte Mutationen im PAH-Gen auf eine Ansprechempfindlichkeit gegenüber Tetrahydrobiopterin hinweisen, andere Mutationen im PAH hingegen nicht.
Die Tetrahydrobiopterin-sensitive Hyperphenylalaninämie kann weiters in einen Tetrahydrobiopterin-sensitiven PAH-Mangel und einen Fehler in der Synthese oder Regeneration von Tetrahydrobiopterin, d.h., typische (schwere) Formen eines Mangels an GTP-Cyclohydrolase 1 (GTPCH), Dihydropteridinreduktase (DHPR) und 6-Pyruvoyltetrahydropterinsynthase (PTPS), unterteilt werden.

Ungefähr 1-3% der Patienten mit hartnäckiger Hyperphenylalaninämie haben eine Defizienz in der Tetrahydrobiopterin-Synthese (Danks et al. J. Inherited Metab. Dis. 1978; 1: 49-53; Berlow et al. Pediatrics. 1980; 65: 837-839). Die Mehrheit der Patienten mit Fehlern in der Tetrahydrobiopterin-Synthese weist häufig Mutationen in jenem Gen auf, das für das phosphateliminierende Enzym PTPS kodiert, welches den zweiten Schritt der Biosynthese von Tetrahydrobiopterin aus Dihydroneopterintriphosphat katalysiert. Solche Patienten, die an einer Tetrahydrobiopterin-sensitiven Hyperphenylalaninämie aufgrund eines mangelhaften Tetrahydrobiopterin-Pathways leiden, welche von einer neurologischen Degeneration begleitet sein kann, sprechen auf eine phenylalaninarme Diätbehandlung nicht an.

Daher sind Behandlungsmaßnahmen erforderlich, welche die tägliche orale Verabreichung von Tetrahydrobiopterin einschließen (Muntau et al., New England Journal of Medicine 2002; 347: 2122-2132). Alternativ wäre auch die Verabreichung von L-Sepiapterin vorstellbar. Niederwieser et al. berichteten, dass die Symptome einer Hyperphenylalaninämie bei einem Patienten mit Dihydrobiopterin-Mangel nach Verabreichung einer einzigen Dosis von L-Sepiapterin vorübergehend verschwanden (Niederwieser et al. Euro J Pediatr 1982; 138(2): 110-2). Demgemäß präsentierten Nichol und Kollegen einen Salvage-Pathway, der für die Regeneration von Tetrahydrobiopterin aus Dihydrobiopterin verantwortlich ist, und zwar über eine Dioxoverbindung und L-Sepiapterin in Niere und Leber, und der von DHPR katalysiert wird (Nichol et al., Proc. Natl. Acad. Sci. 1983; Band 83: 1546-1550). Eine solche Rückgewinnung von Tetrahydrobiopterin scheint unbedingt erforderlich zu sein, um eine stetige Zufuhr von reduziertem Tetrahydrobiopterin sicherzustellen und eine Anhäufung von schädlichen Stoffwechselprodukten zu verhindern. Tatsächlich führen mehrere Mutationen im DHPR-Gen bekanntermaßen zu einer bösartigen Hyperphenylalaninämie (Danks et al. J. Inherit. Metab. Dis. 1978; 1: 49-53).

Die Verabreichung von Tetrahydrobiopterin ist bisher jedoch mit mehreren Problemen verbunden. Feste inerte feuchtigkeitsfreie Formulierungen sind bislang als einzige Option für eine langfristige Lagerung von Tetrahydrobiopterin bekannt, da Tetrahydrobiopterin in Gegenwart eines wässrigen Lösungsmittels rasch abgebaut wird. Das hat zur Folge, dass die individuelle Dosierung, die mit dem Körpergewicht des jeweiligen Patienten in direktem Zusammenhang steht, vom Neugeborenen bis hin zum Erwachsenen stark variiert und mit einer festen Formulierung von Tetrahydrobiopterin, z.B. einem Pulver oder einer Tablette, erzielt werden muss. Dies ist kompliziert und schwierig, da einerseits jede einzelne Pulverdosis separat gewogen werden muss und andererseits das Arzneimittel rasch oxidiert wird, sobald Tetrahydrobiopterin-Pulver Sauerstoff ausgesetzt wird, wenn z.B. der Verschluss der Verpackung geöffnet ist. Folglich war es bislang unmöglich, Tetrahydrobiopterin als Massen-Pulverformulierung zum individuellen Abwiegen durch den Patienten bereitzustellen. Alle Bestrebungen, dieses Problem zu lösen, konzentrieren sich auf Strategien zur Verbesserung der Stabilität von Tetrahydrobiopterin in einer trockenen festen Formulierung und zum Auflösen von verschlossenen festen Single-Unit- Formulierungen, unmittelbar vor der Verabreichung.

Die derzeitigen Strategien zur Überwindung dieser pharmazeutischen und technologischen Herausforderungen konzentrieren sich zum Beispiel auf stabilere Derivate von Tetrahydrobiopterin, stabilere feste Kristallformen (siehe z.B. WO 2005/065018), auf das unter Stickstoff erfolgende Versiegeln in Ampullen und die Lagerung bei ≤ -20°C (Schricks Laboratories, Schweiz) sowie auf das Hinzufügen von Antioxidationsmitteln zu festen Formulierungen (Fiege et al., Mol Genet Metab. 2004 Jan; 81(1): 45-51). Das Nutzen all dieser Strategien verleiht den Multi-Unit-Abgabesystemen allerdings nur für einige Wochen Stabilität, sofern sie nicht bei -20°C oder darunter gelagert werden. Daher ist eine geeignete Formulierung für eine vereinfachte, individuell angepasste orale Behandlung mit Tetrahydrobiopterin erforderlich.

Darreichungsformen von Tetrahydrobiopterin oder eines metabolischen Vorläufers von Tetrahydrobiopterin sind beispielsweise aus WO2006/120176, EP 0 209 689, EP 0 906 913, JP 10 338 637, oder JP 63 267 781 bekannt.

Es wurde nun überraschenderweise eine pharmazeutische Darreichungsform für die orale Verabreichung von Tetrahydrobiopterin oder einen metabolischen Vorläufer davon gefunden, mit deren Hilfe es leicht möglich ist, Tetrahydrobiopterin oder einen metabolischen Vorläufer davon entsprechend dem Gewicht des einzelnen Patienten zu dosieren, die zur Darreichung für Neugeborene, Kindern oder Erwachsene geeignet ist, und in der Tetrahydrobiopterin oder ein metabolischer Vorläufer davon in stabiler Form vorliegt.

In einem Aspekt stellt die vorliegende Erfindung eine orale pharmazeutische Darreichungsform, umfassend Tetrahydrobiopterin, oder einen metabolischen Vorläufer davon, zur Verfügung, die dadurch gekennzeichnet ist, dass sie eine wässrige Lösung von Tetrahydrobiopterin, oder eines metabolischen Vorläufers davon, und eines Antioxidans in Form einer Sulfhydrylverbindung im Molverhältnis 1,5:1 bis 1:4 oder mehr, insbesondere 1:1 bis 1:1,5, in einem Abgabebehälter und eine Dosiereinrichtung zur dosierten Abgabe der wässrigen Lösung umfasst. Im Folgenden steht der Begriff "Antioxidans" immer für ein Antioxidans in Form einer Sulfhydrylverbindung, wobei das Molverhältnis des Tetrahydrobiopterin oder des metabolischen Vorläufers davon zum Antioxidans 1,5:1 bis 1:4 oder mehr, insbesondere von 1:1 bis 1:1,5 beträgt.

Eine orale pharmazeutische Darreichungsform, die gemäß vorliegender Erfindung zur Verfügung gestellt wird, wird hierin auch als "(pharmazeutische) Darreichungsform gemäß (nach) vorliegender Erfindung" bezeichnet.

Eine pharmazeutische Darreichungsform gemäß vorliegender Erfindung umfasst eine orale Darreichungsform zur flüssigen Verabreichung von Tetrahydrobiopterin, oder eines metabolischen Vorläufers davon, in Form einer wässrigen Lösung.

Eine Darreichungsform gemäß vorliegender Erfindung umfasst Tetrahydrobiopterin, oder einen metabolischen Vorläufer davon und das Antioxidans, die gelöst in einem wässrigen Lösungsmittel vorliegen und gegebenenfalls einen oder mehrere pharmazeutisch verträgliche Hilfsstoffe.

Das wässrige Lösungsmittel in einer Darreichungsform gemäß vorliegender Erfindung ist ein Lösungsmittel, das die Protonisierung von darin gelösten Salzen oder Säuren ermöglicht und umfasst Wasser, oder Wasser in Verbindung mit einem weiteren hydrophilen Lösungsmittel, z.B. Ethanol, Isopropanol, Polyethylenglycol, Glycerin, Dimethylsulfoxid, oder Kombinationen davon.

Vorzugsweise umfasst eine wässrige Lösung in einer Darreichungsform gemäß vorliegender Erfindung Wasser als Lösungsmittel, bevorzugt mindestens 50%, mindestens 60%, mindestens 70%, mindestens 80%, mindestens 90%, mindestens 99% Wasser; besonders bevorzugt ist 100% Wasser als Lösungsmittel. Falls der verwendete metabolische Vorläufer von Tetrahydrobiopterin eine geringe Löslichkeit in Wasser hat, als z.B. L-Sepiapterin, das eine Löslichkeit von 0,17 g/100 g H₂O in reinem Wasser aufweist, wird bevorzugt, dass Mischungen von Wasser mit einem weiteren hydrophilen Lösungsmittel, wie z.B. Propylenglycol oder Glycerin, zum Verbessern der Löslichkeit verwendet werden. Es ist auch möglich, die Löslichkeit durch verschiedene Verfahren zu verbessern, welche die Löslichkeit bekanntermaßen verbessern, einschließlich z.B. der Ultrabeschallung.

Tetrahydrobiopterin (5,6,7,8-Tetrahydro-biopterin) in einer Darreichungsform gemäß vorliegender Erfindung ist eine Verbindung der Formel 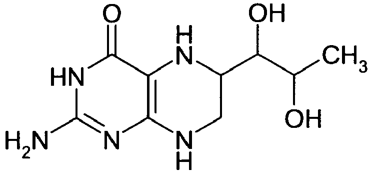 Tetrahydrobiopterin
und entspricht der reduzierten Form von Biopterin. Die chemische Reduktion von Biopterin ergibt zwei Diastereoisomere, nämlich (6S)- und (6R)-5,6,7,8-Tetrahydro-L-biopterin, wobei (6R)-5,6,7,8-Tetrahydro-L-biopterin der natürlichen Form von Tetrahydrobiopterin entspricht. Tetrahydrobiopterin ist auch unter dem Namen Sapropterin oder BH₄ bekannt. (6R)-5,6,7,8-Tetrahydro-L-biopterin in der Form eines Dihydrochloridsalzes wird unter dem Handelsnamen Kuvan® vermarktet. (6R,S)-5,6,7,8-Tetrahydro-L-biopterin im Verhältnis von (6R)/(6S) = 70/30 ist, in der Form eines Dihydrochlorids, erhältlich z.B. von Schircks Laboratories, Schweiz.

Ein metabolischer Vorläufer von Tetrahydrobiopterin in einer Darreichungsform gemäß vorliegender Erfindung schließt eine Pterin- oder Biopterin-Zwischenverbindung ein, welche intrazellulär direkt in 5,6,7,8-Tetrahydrobiopterin oder eine andere Pterin- oder Biopterin-Zwischenverbindung umgewandelt/reduziert werden kann, und zwar entweder über den *de novo-*Weg für die Biosynthese von Tetrahydrobiopterin oder über den für die Regeneration von Tetrahydrobiopterin verantwortlichen Salvage-Pathway. Ein metabolische Vorläufer des (natürlichen) Tetrahydrobiopterin, nämlich L-Sepiapterin der Formel 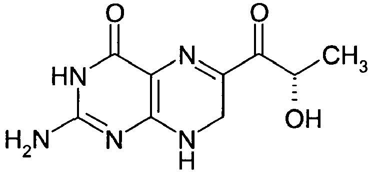 L-Sepiapterin
(CAS 17094-01-8) kann bei Verabreichung durch den Pterin-Salvage-Pathway, der von der Sepiapterinreduktase-Aktivität (=7,8-Dihydro-biopterin:NADP⁺-Oxidoreduktase) abhängt, intrazellulär in (6R)-5,6,7,8-Tetrahydro-L-biopterin umgewandelt werden (Blau et al., Mol Genet Metab. 2001;74(1-2):172-185). Der metabolische Vorläufer des Tetrahydrobiopterins kann in der Form eines Salzes vorliegen, z.B. ein Salz wie jenes des (6S)- und (6R)-5,6,7,8-Tetrahydro-L-biopterins.

In einer Darreichungsform gemäß vorliegender Erfindung ist Tetrahydrobiopterin bevorzugt ausgewählt aus der Gruppe bestehend aus (6R)-5,6,7,8-Tetrahydro-L-biopterin und (6R,S)-5,6,7,8-Tetrahydro-L-biopterin, z.B. in einem Verhältnis (6R)/(6S) = 70/30, und deren Salzen, insbesondere Säureadditionssalzen, beispielsweise Hydrochloridsalzen, wie Dihydrochloridsalzen; besonders bevorzugt ist (6R)-5,6,7,8-Tetrahydro-L-biopterin, bevorzugt in der Form eines Salzes, wie eines Dihydrochloridsalzes. Ein metabolischer Vorläufer von Tetrahydrobiopterin gemäß vorliegender Erfindung ist bevorzugt L-Sepiapterin, z.B. in der Form eines Salzes.

Ein Antioxidans in einer Darreichungsform gemäß vorliegender Erfindung umfasst pharmazeutisch akzeptable Sulfhydrylverbindungen, also Verbindungen, die eine oder mehrere Sulfhydrylgruppen aufweisen, z.B. Sulfhydrylgruppen, die zu kovalenten, brückenbildenden Disulfidbindungen oxidiert werden können. Bevorzugte Beispiele solcher Verbindungen umfassen Aminosäuren oder Aminosäurederivate, die Sulfhydrylgruppen aufweisen, also Sulfhydrylverbindungen, insbesondere Cystein-Verbindungen, z.B. umfassend Cystein, z.B. L-Cystein und davon abgeleitete Verbindungen, wie beispielsweise N-Acetylcystein, Homocystein, N-Acetylhomocystein, Cysteinmethylester, Cysteinethylester, Homocysteinmethylester oder Homocysteinethylester; oder Mischungen aus zwei oder mehreren verschiedenen Sulfhydrylverbindungen, gegebenenfalls in der Form eines Salzes. Ein bevorzugtes Antioxidans in einer Darreichungsform gemäß vorliegender Erfindung ist Cystein, wie L-Cystein, oder N-Acetylcystein, z.B. in der Form eines Salzes, wie eines Hydrochlorids.

Das Molverhältnis von Tetrahydrobiopterin oder des metabolischen Vorläufers davon zum Antioxidans, wobei das Antioxidans eine Sulfhydrylverbindung ist, in einer Darreichungsform gemäß vorliegender Erfindung umfasst ein Verhältnis von 1,5:1 bis 1:4 oder mehr, z.B. 1,1:1; 1:1; 1:1,1; 1:1,2; 1:1,3; 1:1,4; 1:1,5; 1:2; 1:3 oder 1:4; oder mehr; bevorzugt beträgt das Molverhältnis mindestens 1:1, wie z.B. 1:1 bis 1:4, z.B. 1:1 bis 1:1.5, bevorzugt etwa 1:1. Die obere Grenze des in der Darreichungsform gemäß vorliegender Erfindung enthaltenen Antioxidans, wobei das Antioxidans eine Sulfhydrylverbindung ist, wird durch die Löslichkeit des jeweiligen Antioxidans im jeweils verwendeten wässrigen Lösungsmittel festgelegt.

Eine Stabilisierung des Tetrahydrobiopterins oder eines metabolischen Vorläufers davon in einer Darreichungsform gemäß vorliegender Erfindung wird bereits bei einem Molverhältnis von 1,5:1 (Tetrahydrobiopterin:Antioxidans), eine beträchtliche Stabilisierung bei einem Molverhältnis von (etwa) 1:1 beobachtet.

Im Fall von (6R)-5,6,7,8-Tetrahydro-L-biopterin in der Form eines Dihydrochlorids als Tetrahydrobiopterin und L-Cystein in der Form eines Hydrochlorids als Antioxidans werden bevorzugt 1 g Antioxidans auf 1,5 g bis 2 g, z.B. auf 1,6 g, 1,7 g, 1,8 g, 1,9 g oder 2,0 g Tetrahydrobiopterin eingesetzt, z.B. 1 g Antioxidans auf 2,0 g Tetrahydrobiopterin.

Die bevorzugte Konzentration von Tetrahydrobiopterin und/oder eines Vorläufers davon in einer Darreichungsform gemäß vorliegender Erfindung sollte die Verabreichung von genügend Tetrahydrobiopterin und/oder eines metabolischen Vorläufers davon ermöglichen. Die Flüssigkeitsmenge soll genügend Tetrahydrobiopterin und/oder einen metabolischen Vorläufer davon umfassen, um bei einem an Tetrahydrobiopterin-sensitiver Hyperphenylalaninämie leidenden Patienten eine therapeutische Reaktion auszulösen. Bei einer speziellen Ausführungsform der vorliegenden Erfindung wird die exakte Dosierung durch Überwachung der Wirksamkeit bestimmt, um die Symptome von Tetrahydrobiopterin-sensitiver Hyperphenylalaninämie zu lindern, welche bei Patienten mit einer Tetrahydrobiopterin-Synthese-Defizienz z.B. an normalen physiologischen Phenylalaninspiegeln und einer normalen Produktion von Catecholaminen und Serotonin erkennbar sind. Die erforderliche Dosis liegt typischerweise im Bereich zwischen 5 mg und 80 mg Tetrahydrobiopterin und/oder eines metabolischen Vorläufers davon pro kg Körpergewicht, vorzugsweise im Bereich von 10 mg bis 40 mg pro kg Körpergewicht. Diese Dosis kann in einer einzelnen Dosis oder in mehreren kleineren Dosierungen verabreicht werden, welche im Laufe des Tages, insbesondere in zwei Dosen von 2,5 mg bis 40 mg Tetrahydrobiopterin und/oder eines metabolischen Vorläufers davon, pro kg Körpergewicht, vorzugsweise von 5 mg bis 20 mg pro kg Körpergewicht bei jeder Dosierung, verabreicht werden. Es wird besonders bevorzugt, dass 10 mg/kg Körpergewicht zweimal täglich verabreicht werden, und somit ist es bei einem Neugeborenen von z.B. 4 kg erforderlich, dass etwa 40 mg Tetrahydrobiopterin in einer festgelegten Menge der flüssigen Formulierung enthalten sind, während bei einem Erwachsenen von 100 kg etwa 1000 mg Tetrahydrobiopterin in einer festgelegten Menge der flüssigen Formulierung enthalten sein sollen.

Eine Darreichungsform gemäß vorliegender Erfindung umfasst Tetrahydrobiopterin oder einen metabolischen Vorläufer davon, insbesondere (6R)-5,6,7,8-Tetrahydro-L-biopterin-2HCl und/oder einen metabolischen Vorläufer davon, im Bereich von 0,5 Gew% bis 50,0 Gew%, vorzugsweise 2,0 Gew% bis 25 Gew.%, wie 2,0 Gew% bis 20 Gew.%, noch bevorzugter von 4,0 Gew% bis 15 Gew%, z. B. 5,0 Gew% bis 15 Gew%, beispielsweise etwa 10 Gew%. Die Konzentration des Antioxidans in einer Darreichungsform gemäß vorliegender Erfindung wird angepasst, um ein ausreichendes Molverhältnis zur Stabilisierung des Tetrahydrobiopterins zu erhalten.

Die wässrige Lösung in einer Darreichungsform gemäß vorliegender Erfindung weist bevorzugt einen sauren pH-Wert auf, beispielsweise einen pH Wert unterhalb von 5, vorzugsweise unterhalb von 3 und noch bevorzugter unterhalb von 2, wie etwa 5,0, 4,5, 4,0, 3,5, 3,0, 2,5, 2,0, 1,5, 1,0 oder weniger, z.B. 1.0 bis 5.5. Der gewünschte "Säuregrad" wird typischerweise allein durch Auflösen von Tetrahydrobiopterin-Säureadditionssalzen, z.B. (6R)-5,6,7,8-Tetrahydro-L-biopterin-dihydrochlorid-Säureadditionssalz, erzielt. Falls die gewünschten niedrigen pH-Werte nicht durch das jeweilige Tetrahydrobiopterin selbst erzielt werden, wird eine Anpassung des gewünschten pH-Werts mittels einer ansäuernden Substanz bevorzugt. Der gewünschte saure pH-Wert unterhalb von 2 kann bei einer L-Sepiapterin enthaltenden, sauren flüssigen Formulierung, die in einer zu 99% reinen kristallinen Form erhältlich ist (Cayman Chemical), zum Beispiel durch Ergänzung mit einer geeigneten Menge einer ansäuernden Substanz eingestellt werden.

Eine pharmazeutische Darreichungsform gemäß vorliegender Erfindung kann weiterhin einen oder mehrere pharmazeutisch verträgliche Hilfsstoffe umfassen. Pharmazeutisch verträgliche Hilfsstoffe die in einer Darreichungsform gemäß vorliegender Erfindung vorliegen können, umfassen Hilfsstoffe, die in der Galenik Verwendung finden, bevorzugt. Geschmackszusätze, Konservierungsmittel, ansäuernde Substanzen und/oder Farbmittel.

Eine pharmazeutische Darreichungsform zur oralen Verabreichung gemäß vorliegender Erfindung kann durch Zusatz eines wässrigen Lösungsmittels zu einer festen pharmazeutischen Zusammensetzung, umfassend Tetrahydrobiopterin, oder einen metabolischen Vorläufer davon, ein Antioxidans, wobei das Antioxidans eine Sulfhydrylverbindung ist und wobei das Molverhältnis des Tetrahydrobiopterin zum Antioxidans 1,5:1 bis 1:4 oder mehr, insbesondere von 1: 1 bis 1: 1,5 beträgt, und gegebenenfalls pharmazeutisch verträgliche Hilfsstoffe, oder durch die Herstellung einer wässrigen Lösung von Tetrahydrobiopterin, oder eines metabolischen Vorläufers davon und eines Antioxidans, gegebenenfalls unter Zusatz eines pharmazeutisch verträglichen Hilfsstoffs, wobei die wässrige Lösung in einen Abgabebehälter, der mit eine Dosiereinrichtung zur dosierten Abgabe der wässrigen Lösung verbunden werden kann, oder verbunden ist, eingebracht ist oder eingebracht wird, erhalten werden, wobei der Wassergehalt einer festen pharmazeutischen Zusammensetzung vorzugsweise im Bereich von 1,0% bis 5,0%, z.B. 1,5% bis 4,0% liegt.

Eine solche feste pharmazeutische Zusammensetzung umfasst beispielsweise Pulver, Pellets Granulate oder Preßlinge, wie Tabletten, in denen, neben Geschmackszusätzen, Konservierungsmitteln, ansäuernden Substanzen und/oder Farbmitteln als pharmazeutisch verträgliche Hilfsstoffe weitere pharmazeutisch verträgliche Hilfsstoffe, wie Trocknungsmittel, Füllstoffe und/oder Granuliermittel vorliegen können. Werden solche festen pharmazeutischen Zusammensetzungen zur Herstellung einer Darreichungsform gemäß vorliegender Erfindung verwendet, kann die wässrige Lösung in einer Darreichungsform gemäß vorliegender Erfindung ebenfalls solche weiteren pharmazeutisch verträglichen Hilfsstoffe enthalten.

Ein Geschmackszusatz gemäß vorliegender Erfindung umfasst eine Verbindung, mit deren Hilfe der Geschmack einer pharmazeutischen Formulierung gemäß vorliegender Erfindung verbessert werden kann, wie beispielsweise natürlich vorkommende Süßstoffe, künstliche Nahrungssüßstoffe, Geschmacksstoffe, die beispielsweise einen Zimt-, Vanille-, Kirsch-, Erdbeer-, Orangen-, Bananen- und/oder Apfelgeschmack hervorrufen können.

Ein natürlich vorkommender Süßstoff, der in einer Darreichungsform gemäß vorliegender Erfindung Verwendung finden kann, umfasst Monosaccharide, wie Glucose, Galactose, Mannose, Xylitol; Disaccharide, wie Fructose, Saccharose, Lactose, Maltose, Mischungen von Mono- und Disacchariden, sowie Mischungen von Mono- und/oder Disacchariden mit Polysacchariden, beispielsweise Maltodextrose, ein künstlicher Nahrungssüßstoff schließt kalorienfreie oder kalorienarme Zuckerersatzstoffe, wie Sorbit, Mannit, Aspartam (z.B. Nutrasweet™, Equal™), Saccharin, Acesulfam K, ein.
Ein Süßstoff, der in einer Darreichungsform gemäß vorliegender Erfindung vorliegen kann, umfasst vorzugsweise Glucose, Galactose, Mannose, Xylitol, Fructose, Saccharose, Lactose, Maltose und/oder Maltodextrin.

Ein Konservierungsmittel, das in einer Darreichungsform gemäß vorliegender Erfindung Verwendung finden kann, umfasst alle natürlichen oder synthetischen Verbindungen, die pharmazeutisch zugelassen sind und Lebensmitteln, Getränken oder Pharmazeutika beigefügt werden können, um den Verderb, sei es durch Mikrobenwachstum oder durch unerwünschte chemische Veränderungen, hinauszuzögern, wie antimikrobielle Verbindungen, die das Wachstum von Bakterien und Pilzen hemmen, z.B. Benzoate, Borate, Sorbate, Carbonate, Acetate, wie. Natriumbenzoat, Kaliumbenzoat, Benzoesäure, Natriumsorbat, Kaliumsorbat, Sorbinsäure, p-Hydroxybenzoesäuremethylester, p-Hydroxybenzoesäureethylester, Natriumborate, Dimethyldicarbonat oder Dimethylacetat. Andere Konservierungsmittel können zusätzlich auch eine antioxidierende Wirkung aufweisen und zur Stabilisierung von Tetrahydrobiopterin in einer Darreichungsform gemäß vorliegender Erfindung beitragen. Konservierungsmittel, die eine antioxidierende Wirkung aufweisen, umfassen z.B. Phenolverbindungen, wie z.B. butyliertes Hydroxyanisol (BHA) und die verwandte Verbindung butyliertes Hydroxytoluol (BHT).

Ein Konservierungsmittel in einer Darreichungsform gemäß vorliegender Erfindung umfasst vorzugsweise Natriumbenzoat, Natriumborat, Dimethyldicarbonat, Dimethylacetat, butyliertes Hydroxyanisol, oder butyliertes Hydroxytoluol.

Eine ansäuernde Substanz, die in einer Darreichungsform gemäß vorliegender Erfindung vorliegen kann, umfasst pharmazeutisch zugelassene, salzbildende Säuren, siehe z.B. Berge et al., Pharmaceutical salts 1977 J.Pharm.Sci.; 66(1):1-19, z.B. feste Säuren. Eine ansäuernde Substanz gemäß vorliegender Erfindung kann auch eine antioxidierende Wirkung aufweisen und somit die Stabilität einer Darreichungsform gemäß vorliegender Erfindung noch weiter verbessern. Beispiele solcher ansäuernder Substanzen umfassen Zitronensäure, Weinsäure, Benzoesäure.

Eine wässrige Lösung enthaltend Tetrahydrobiopterin und/oder einen metabolischen Vorläufer davon, gemäß vorliegenden Ansprüche, kann ihre Farbe bei einer Lagerung über einen längeren Zeitraum gegebenenfalls verändern, wobei eine solche Verfärbung nicht unbedingt bedeutet, dass die therapeutische Wirksamkeit beeinträchtigt wird. Beispielsweise um das äußere Erscheinungsbild der wässrigen Lösung zu bewahren, kann eine Darreichungsform gemäß vorliegender Erfindung ein Farbmittel umfassen.

Ein Farbmittel das in einer Darreichungsform gemäß vorliegender Erfindung vorliegen kann, schließt natürliche und synthetische Farbstoffe, die pharmazeutisch akzeptabel sind, ein. Eine große Anzahl solcher Farbmittel ist bekanntermaßen für die Verwendung in pharmazeutischen Zusammensetzungen geeignet, beispielsweise natürliche Farbstoffe, wie Annatto-Extrakt, Anthocyaninen, ß-Carotin, beta-APO 8, Carotinal, schwarzer Johannisbeere, gebrannter Zucker, Canthaxanthin, Karamell, Carbo medicinalis, Karmin, Karminblau, Carminsäure, Karotte, Chlorophyll, Chlorophyllin, Cochenille-Extrakt, Kupfer-Chlorophyll, Kupfer-Chlorophyllin, Curcumin, Curcumin/CU-Chlor, Holunderbeere, Weinbeere, Hibiskus, Lutein, gemischte Carotinoide, Paprika, Riboflavin, Spinat, Brennnessel, Titandioxid, Gelbwurz, Naturfarben, Aronia/Rotfrucht, Rübensaftfarben, Paprikaextrakt, Paprika-Oleoresin; oder synthetische Farbstoffe, wie Allurarot, Amaranth, Carmoisin, Echtrot E, Erythrosin, Grün S, Patentblau V, Ponceau 4R, Chinolingelb, Rot 2G, Sunsetgelb, Tartrazin.

Ein Abgabebehälter in einer Darreichungsform gemäß vorliegender Erfindung umfasst einen luft- und flüssigkeitsdichten Behälter, vorzugsweise aus Glas, z.B. Braunglas, oder Kunststoff, z.B. optisch undurchsichtigen Kunststoff. Der Abgabebehälter weist bevorzugt ein Fassungsvermögen von 10 ml bis 1000 ml, wie 25 ml bis 1000 ml, z. B. 25 ml bis 500 ml, wie 50 ml bis 200 ml, beispielsweise 10 ml, 25 ml, 50 ml, 75 ml, 100 ml, 125 ml, 150 ml, 175 ml, 200 ml, 300 ml, 400 ml, 500 ml, 600 ml, 700 ml, 800 ml, 900 ml oder 1000 ml; auf.

Der Abgabebehälter in einer Darreichungsform gemäß vorliegender Erfindung kann Befestigungsmittel, z.B. Schraubengewinde, die zum Verbinden, z.B. zum Befestigen, der Dosiereinrichtung, die zur dosierten Abgabe der wässrigen Lösung geeignet ist, mit oder an dem Abgabebehälter umfassen.

Eine Dosiereinrichtung zur dosierten Abgabe der wässrigen Lösung in einer Darreichungsform gemäß vorliegender Erfindung umfasst eine Flüssigkeitsdosiereinrichtung, z.B. eine Pumpe, Spritze oder Pipettenpumpe, die zum Abgeben gewünschter Mengen einer wässrigen Lösung geeignet ist.

Die Dosiereinrichtung umfasst vorzugsweise einen Betätiger, der im Abgabebehälter angeordnet sein kann oder sich in den Abgabebehälter erstrecken kann. Die Dosiereinrichtung umfasst vorzugsweise eine Pumpvorrichtung als Betätiger, welche am Abgabebehälter beweglich montiert sein kann und sich z.B. über eine Kanüle, in den Abgabebehälter erstreckt. Durch Bewegen der Pumpvorrichtung zur Auslassöffnung des Abgabebehälters tritt eine genau dosierte Menge der wässrigen Lösung aus der Auslassöffnung des Abgabebehälters.

Die Dosiereinrichtung ist zur Abgabe von Aliquoten der wässrigen Lösung geeignet, z.B. von 100 µl bis 2000 µl, wie 200 µl bis 1500 µl, z.B. 500 µl bis 1000 µl; beispielsweise100 µl, 200 µl, 300 µl, 400 µl, 500 µl, 600 µl, 700 µl, 800 µl, 900 µl, 1000 µl, 1100 µl, 1200 µl, 1300 µl, 1400 µl, 1500 µl, 1600 µl, 1700 µl, 1800 µl, 1900 µl oder 2000 µl. Das exakte Volumen der durch die Dosiereinrichtung abgegebenen Aliquote der wässrigen Lösung kann entweder vorgegeben sein oder an das gewünschte Volumen mechanisch anpassbar sein.

In einer Ausführungsform stellt die vorliegende Erfindung eine Darreichungsform gemäß vorliegender Erfindung zur Verfügung, in der die Dosiereinrichtung mit dem Abgabebehälter fest verbunden ist.

Der Abgabebehälter mit oder ohne der Dosiereinrichtung kann zusätzlich in eine aus Aluminium-Verbundmaterial bestehende Folie eingeschweißt sein, um eine Oxidation des Wirkstoffes vor der Verwendung zu verhindern.

In einer anderen Ausführungsform stellt die vorliegende Erfindung einen Kit zur Verfügung, der die wässrige Lösung in einem Abgabebehälter und die Dosiereinrichtung getrennt, aber zusammen in einer Packung verpackt, umfasst.

Eine wässrige Lösung in einer Darreichungsform gemäß vorliegender Erfindung, kann mit Hilfe einer Dosiereinrichtung zur dosierten Abgabe, die mit dem Abgabebehälter verbunden wird, oder verbunden ist, in passender Dosis einem Patienten verabreicht werden. Die Verabreichung kann durch direkte Einnahme der passenden Dosis durch den Patienten erfolgen, oder die passende Dosis kann, möglichst unmittelbar vor dem Verzehr, einem Nahrungsmittel, wie Milch, Joghurt, alkoholfreie Getränke, Säfte, z.B. Orangensaft oder Apfelsaft, Coca Cola, Suppe, Wasser, Babynahrung zur Einnahme durch den Patienten beigefügt werden. Es hat sich beispielsweise herausgestellt, dass Verdünnungen einer wässrigen Lösung in einer Darreichungsform gemäß vorliegender Erfindung mit Muttermilch oder Verdünnungen einer wässrigen Lösung in einer Darreichungsform gemäß vorliegender Erfindung mit einer Lösung einer Trockenmilchnahrung, z.B. einer 12.2 Gew%igen Lösung einer Trockenmilchnahrung, wie Aptamil®, HA Pre®, Milupa®, nicht koagulieren; z.B. wenn ein Verhältnis von 1 Teil einer wässrigen Lösung in einer Darreichungsform gemäß vorliegender Erfindung enthaltend z.B. 10% Tetrahydrobiopterin, mit 5 Teilen von Muttermilch oder mit 5 Teilen einer Verdünnung einer wässrigen Darreichungsform mit einer Lösung einer Trockenmilchnahrung, z.B. einer 12.2 Gew%igen Lösung einer Trockenmilchnahrung, wie Aptamil®, HA Pre®, Milupa®, eingesetzt wird. An Kinder und Säuglinge kann eine wässrige Lösung in einer Darreichungsform gemäß vorliegender Erfindung daher auch zusammen mit Muttermilch oder mit einer Lösung einer Trockenmilchnahrung, wie Aptamil®, HA Pre®, Milupa®, oder auch mit Babynahrung, wie sie z.B. käuflich zu erwerben ist, verabreicht werden.

In einem Aspekt kann die Herstellung einer Darreichungsform gemäß vorliegender Erfindung durch die Herstellung einer wässrigen Lösung von Tetrahydrobiopterin, oder eines metabolischen Vorläufers davon und eines Antioxidans, gegebenenfalls unter Zusatz von Arzneimittelhilfsstoffen, erfolgen, wobei die wässrige Lösung in einen Abgabebehälter, der mit einer Dosiereinrichtung zur dosierten Abgabe der wässrigen Lösung verbunden ist oder verbunden wird, eingebracht ist oder eingebracht wird.

Das Herstellungsverfahren für eine Darreichungsform gemäß vorliegender Erfindung umfasst in einem Aspekt die folgenden Schritte:
(i) das Abwiegen eines Antioxidans, wobei das Antioxidans eine Sulfhydrylverbindung ist, und von Tetrahydrobiopterin oder eines metabolischen Vorläufers davon und gegebenenfalls pharmazeutisch verträglichen Hilfsstoffen,
(ii) gegebenenfalls das Vermischen von Ingredienzien aus (i),
(iii) das Auflösen des Antioxidans, Tetrahydrobiopterins oder eines metabolischen Vorläufers davon und gegebenenfalls pharmazeutisch verträglicher Hilfsstoffe, oder Mischungen davon in einem wässrigen Lösungsmittel, und
(iv) gegebenenfalls das Einbringen der wässrigen Lösung oder der wässrigen Lösungen aus (iii) in einen Abgabebehälter, der mit einer Dosiereinrichtung zur dosierten Abgabe der wässrigen Lösung verbunden ist oder verbunden wird.

Das Auflösen des Antioxidans, Tetrahydrobiopterins oder eines metabolischen Vorläufers davon gemäß vorliegender Erfindung und gegebenenfalls pharmazeutisch verträglicher Hilfsstoffe kann entweder durch jeweils getrennte Auflösung der Ingredienzien in einem wässrigen Lösungsmittel erfolgen, oder ein oder mehrere, gegebenenfalls alle, Ingredienzien werden gemeinsam in einem wässrigen Lösungsmittel gelöst.

Das Einbringen der wässrigen Lösung oder der wässrigen Lösungen in einen Abgabebehälter, der mit einer Dosiereinrichtung zur dosierten Abgabe der wässrigen Lösung verbunden ist oder verbunden wird, kann durch Abfüllen einer wässrigen Lösung oder wässriger Lösungen von einem, mehreren oder allen Ingredienzien in den Abgabebehälter erfolgen, oder eine wässrige Lösung von einem, mehreren, oder allen Ingredienzien wird direkt im Abgabebehälter erzeugt und gegebenenfalls werden wässrige Lösungen von einem oder mehreren Ingredienzien hinzugefügt.

Wenn die feste Darreichungsform z.B. ein Pulver ist, umfasst ein Herstellungsverfahren z.B. die folgenden Schritte:
(i) das Abwiegen eines Antioxidans und von Tetrahydrobiopterin oder eines metabolischen Vorläufers davon und, falls gewünscht, von pharmazeutisch verträglichen Hilfsstoffen,
(ii) das Vermischen der Ingredienzien aus, wobei das Molverhältnis des Tetrahydrobiopterin zum Antioxidans 1,5:1 bis 1:4 oder mehr, insbesondere von 1: 1 bis 1: 1,5 beträgt,
(iii) das Auflösen der Mischung aus (ii) in einem wässrigen Lösungsmittel, gegebenenfalls in einen Abgabebehälter, der mit einer Dosiereinrichtung zur dosierten Abgabe der wässrigen Lösung verbunden ist oder verbunden wird, und
(iv) gegebenenfalls das Einbringen der wässrigen Lösung aus (iii) in einen Abgabebehälter, der mit einer Dosiereinrichtung zur dosierten Abgabe der wässrigen Lösung verbunden ist oder verbunden wird.

Wenn die feste Darreichungsform gemäß vorliegender Erfindung z.B. im luftdichten Behälter, beispielsweise in Sachets, in Form eines Granulats vorliegt, umfasst ein Herstellungsverfahren z.B. die folgenden Schritte:
(i) das Abwiegen von pharmazeutisch verträglichen Hilfsstoffen, eines Antioxidans und von Tetrahydrobiopterin und/oder eines metabolischen Vorläufers davon,
(ii) das Vermischen der Ingredienzien aus (i),
(iii) das Granulieren der Mischung aus (ii) mit einem Granuliermittel,
(iv) eine Druck- oder Wärmetrocknung der Granule aus (iii),
(v) gegebenenfalls und vorzugsweise eine Größeneinstellung der Granule aus (iv), z.B. durch Siebung,
(vi) gegebenenfalls Hinzufügen und Einmischen von weiteren pharmazeutisch verträglichen Hilfsstoffen zu Granulen aus (v) und,
(vii) das Auflösen der Mischung aus (vi) in einem wässrigen Lösungsmittel, gegebenenfalls in einem Abgabebehälter, der mit einer Dosiereinrichtung zur dosierten Abgabe der wässrigen Lösung verbunden ist oder verbunden wird, und
(viii) gegebenenfalls das Einbringen der wässrigen Lösung aus (vii) in einen Abgabebehälter, der mit einer Dosiereinrichtung zur dosierten Abgabe der wässrigen Lösung verbunden ist oder verbunden wird.

Wenn die feste Zusammensetzung z.B. in Form von Pellets vorliegt, umfasst ein Herstellungsverfahren z.B. die folgenden Schritte:
(i) das Abwiegen von pharmazeutisch verträglichen Hilfsstoffen, eines Antioxidans und von Tetrahydrobiopterin und/oder eines metabolischen Vorläufers davon,
(ii) das Vermischen der Ingredienzien aus (i),
(iii) das Granulieren der Mischung aus (ii) mit einem Granuliermittel,
(iv) das Pelletieren der Granule aus (iii),
(v) das Trocknen der Pellets aus (iv),
(vi) das Auflösen der Pellets aus (v) in einem wässrigen Lösungsmittel, gegebenenfalls in einem Abgabebehälter, der mit einer Dosiereinrichtung zur dosierten Abgabe der wässrigen Lösung verbunden ist oder verbunden wird, und
(vii) gegebenenfalls das Einbringen der wässrigen Lösung aus (vi) in einen Abgabebehälter, der mit einer Dosiereinrichtung zur dosierten Abgabe der wässrigen Lösung verbunden ist oder verbunden wird.

Es hat sich gezeigt, dass eine wässrige Lösung in einer Darreichungsform gemäß vorliegender Erfindung bei einer Temperatur von 2°C bis 60°C, wie 4°C bis 40°C, z.B. bei Raumtemperatur stabil ist.

Eine wässrige Lösung in einer Darreichungsform gemäß vorliegender Erfindung wird hierin als "stabil" bezeichnet, wenn sie mindestens 95%, z.B. 96%, 97%, 98% oder 99% der Anfangskonzentration von Tetrahydrobiopterin, oder eines metabolischen Vorläufers davon, insbesondere (6R)-5,6,7,8-Tetrahydro-L-biopterin-2HCl, nach einer Lagerung von mindestens einem Monat, z.B. von 2, 3, 4, 5, 6, 9 oder 12 Monaten, enthält. Die Anfangskonzentration von Tetrahydrobiopterin, oder eines metabolischen Vorläufers davon, wird dabei als jene Konzentration definiert, die unmittelbar nach der Bereitstellung der wässrigen Lösung in einer Darreichungsform gemäß vorliegender Erfindung durch geeignete Mittel, z.B. HPLC, bestimmt wird.

Beispielsweise infolge der Möglichkeit einer hohen Dosiergenauigkeit ist eine Darreichungsform gemäß vorliegender Erfindung besonders geeignet zur Verabreichung von Tetrahydrobiopterin, oder eines metabolischen Vorläufers davon, an Säuglinge oder Kinder, wobei beispielsweise die wässrige Lösung in einer Darreichungsform gemäß vorliegender Erfindung vorteilhafterweise zusammen mit einem Nahrungsmittel verabreicht werden kann, beispielsweise zusammen mit Milch, wie Muttermilch und Milch, hergestellt aus Trockenmilchnahrung, alkoholfreien Getränke, Suppen, Babynahrung.

In einem weiteren Aspekt stellt die vorliegende Erfindung eine pharmazeutische Darreichungsform zur Verfügung, die besonders zur Verabreichung von Tetrahydrobiopterin, oder eines metabolischen Vorläufers davon, an Säuglinge oder Kinder, geeignet ist, z.B. die Verwendung einer pharmazeutischen Darreichungsform gemäß vorliegender Erfindung zur Herstellung eines Arzneimittels zur Behandlung von Phenylketonurie-Varianten, die durch einen Tetrahydrobiopterin-Mangel verursacht werden, insbesondere Hyperphenylalaninämie, durch Verabreichung von Tetrahydrobiopterin oder eines metabolischen Vorläufers davon an Kinder oder Säuglinge, insbesondere durch Verabreichung in Kombination mit Milch oder Babynahrung.

In einem anderen Aspekt offenbart die vorliegende Erfindung die Verwendung eines Antioxidans, wobei das Antioxidans eine Sulfhydrylverbindung ist, zum Stabilisieren einer wässrigen Lösung von Tetrahydrobiopterin, oder eines metabolischen Vorläufers davon, für einen Lagerungszeitraum, z.B. zur Lagerung im offenen Zustand, von mindestens einem Monat, z.B. von 2, 3, 4, 5, 6, 9 oder 12 Monaten, wobei insbesondere das Molverhältnis des Tetrahydrobiopterins oder des metabolischen Vorläufers davon zum Antioxidans ein Verhältnis von etwa 1,5:1 bis 1:4, insbesondere von 1:1 bis 1:1,5 umfasst.

In einem weiteren Aspekt offenbart die vorliegende Erfindung die Verwendung eines Antioxidans, wobei das Antioxidans eine Sulfhydrylverbindung ist, insbesondere eines Cysteins, zum Stabilisieren einer wässrigen Lösung von Tetrahydrobiopterin, oder eines metabolischen Vorläufers davon , wobei das Tetrahydrobiopterin, oder der metabolische Vorläufer davon bei 40°C ± 2°C und 75% ± 5% relativer Feuchtigkeit und/oder bei 25°C ± 2°C bei 60% ± 5% relativer Feuchtigkeit stabil sind, insbesondere wobei 95%, insbesondere 96%, 97%, 97.5%, 98% oder 99% der Anfangskonzentration von Tetrahydrobiopterin, oder eines metabolischen Vorläufers davon nach einer Lagerung von mindestens einem Monat, insbesondere 2, 3, 4, 5, 6, 9 oder 12 Monaten, erhalten bleiben.

Eine wässrige Lösung in einer Darreichungsform gemäß vorliegender Erfindung kann als Medizin, oder für die Produktion einer Medizin, zum Behandeln, Lindern oder Heilen einer Krankheit, welche die Verabreichung von Tetrahydrobiopterin und/oder eines metabolischen Vorläufers davon erfordert, wie z.B. Hyperphenylalaninämie, z.B. Tetrahydrobiopterin-sensitive Hyperphenylalaninämie, Diabetes Typ II, mehrere Formen der Hypertonie, Erektionsstörungen und Untergruppen von Störungen mit einem veränderten Neurotransmitter-Metabolismus, wie z.B. die Parkinson-Krankheit, verwendet werden.

Der Begriff "Hyperphenylalaninämie", wie er hierin verwendet wird, umfasst eine Stoffwechselstörung bei einem Säugetier, vorzugsweise beim Menschen, welche durch erhöhte Blutserumspiegel von Phenylalanin, typischerweise im Bereich von 120 und 600 µmol/l, gekennzeichnet ist.

Der Begriff "Tetrahydrobiopterin-sensitive Hyperphenylalaninämie", wie er hierin verwendet wird, umfasst jene Variante der Hyperphenylalaninämie, die durch einen Phenylalaninhydroxylase-Mangel oder einen Fehler in der Synthese von Tetrahydrobiopterin definiert ist, die durch Verabreichung von Tetrahydrobiopterin und/oder eines Vorläufers davon gelindert, behandelt oder geheilt werden kann.

Die Behandlung von Hyperphenylalaninämie und insbesondere die Behandlung von Tetrahydrobiopterin-sensitiver Hyperphenylalaninämie erfordert eine tägliche Verabreichung einer Gesamtdosis, die vom Körpergewicht des Patienten abhängt. Die täglich erforderliche Gesamtdosis wird im allgemeinen in zwei Portionen verabreicht, kann aber auch in mehr als zwei Portionen verabreicht werden.

### Beschreibung der Figuren

**Fig. 1****.** Einfluss von in den angegebenen Konzentrationen (w/v) verwendetem N-Acetylcystein auf die Stabilität von Tetrahydrobiopterin in einer wässrigen Lösung. Die angegebenen Werte sind Mittelwerte von mindestens 3 Versuchen ± Standardabweichung.
**Fig. 2****.** Einfluss von in den angegebenen Konzentrationen (w/v) verwendetem Cystein auf die Stabilität von Tetrahydrobiopterin in einer wässrigen Lösung. Die angegebenen Werte sind Mittelwerte von mindestens 3 Versuchen ± Standardabweichung.

### Beispiel 1

### Dosierungsparameter für eine Einzelverabreichung einer Darreichungsform gemäß vorliegender Erfindung beim Säugling bis zum Erwachsenen.

Die nachfolgende Tabelle 1 veranschaulicht die exakte Dosierung bei Verwendung einer 10%igen, flüssigen Formulierung von Tetrahydrobiopterin mit Hilfe einer Dosierpumpe zum Abgeben von 500 µl-Aliquoten (um die geeignete Menge an Tetrahydrobiopterin, die zum Behandeln, Lindern oder Heilen einer Tetrahydrobiopterin-sensitiven Hyperphenylalaninämie erforderlich ist, zu verabreichen, muss die beschriebene Dosis jedoch zweimal täglich angewandt werden).

**Tabelle 1: Dosierung 10 mg/kg**

| Gewicht des Patienten | ml pro Dosis | |
|---|---|---|
| 5 kg | → 0,5 ml (1 Einheit) | 10%ige Tetrahydrobiopterin-Lösung |
| 10 kg | → 1 ml (2 Einheiten) | 10%ige Tetrahydrobiopterin-Lösung |
| 20 kg | → 2 ml (4 Einheiten) | 10%ige Tetrahydrobiopterin-Lösung |
| 40 kg | → 4 ml (8 Einheiten) | 10%ige Tetrahydrobiopterin-Lösung |
| 70 kg | → 7 ml (14 Einheiten) | 10%ige Tetrahydrobiopterin-Lösung |
| 100 kg | → 10 ml (20 Einheiten) | 10%ige Tetrahydrobiopterin-Lösung |

Um die Anzahl der Einheiten, die bei Erwachsenen von 70 bis 100 kg und mehr erforderlich sind, zu reduzieren, kann alternativ eine Dosierpumpe eingesetzt werden, die imstande ist, 1,0-, 1,5- oder 2,0 ml-Aliquote abzugeben.

### Beispiel 2

### Wässrige BH₄-Zusammensetzungen

**Flüssige Formulierung A, umfassend 10% w/w BH₄ 2HCl**

| | |
|---|---|
| (6R)-5,6,7,8-Tetrahydro-L-biopterin·2HCl | 10 g |
| L-Cystein-HCl | 5 g |
| Natriumbenzoat (EuAB) | 0,2 g |
| demineralisiertes Wasser (EuAB) | ad 100 ml |

**Flüssige Formulierung B, umfassend 10% w/w BH₄ 2HCl**

| | |
|---|---|
| (6R)-5,6,7,8-Tetrahydro-L-biopterin·2HCl | 10 g |
| L-Cystein-HCl | 5 g |
| Syloid®AL | 5 g |
| Natriumbenzoat (EuAB) | 0,2 g |
| demineralisiertes Wasser (EuAB) | ad 100 ml |

**Flüssige Formulierung AB, umfassend 10% w/w BH₄ 2HCl**

| | |
|---|---|
| (6R)-5,6,7,8-Tetrahydro-L-biopterin·2HCl | 10 g |
| L-Cystein-HCl | 5 g |
| Natriumbenzoat (EuAB) | 0,1 g |
| Kaliumsorbat (EuAB) | 0.1 g |
| demineralisiertes Wasser (EuAB) | ad 100 ml |

### Beispiel 3

### Pulvervormischungen mit Tetrahydrobiopterin zur Herstellung von wäßrigen Lösungen

Die trockenen Formulierungen von Tetrahydrobiopterin sind dazu ausgelegt, nach Wiederherstellung in einer geeigneten Menge von demineralisiertem Wasser 100 ml einer 10%igen flüssigen Tetrahydrobiopterin-Formulierung zu ergeben. Die trockene Formulierung umfasst vorzugsweise ein Trocknungsmittel.

**Feste Zusammensetzung C**

| | |
|---|---|
| (6R)-5,6,7,8-Tetrahydro-L-biopterin·2HCl | 10 g |
| L-Cystein·HCl wasserfrei | 5 g |
| Natriumbenzoat (EuAB) | 0,2 g |

**Feste Zusammensetzung D (mit 1% Syloid®)**

| | |
|---|---|
| (6R)-5,6,7,8-Tetrahydro-L-biopterin·2HCl | 10 g |
| L-Cystein·HCl wasserfrei | 5 g |
| Syloid®AL 1 FP (5%) | 0,15 g |
| Natriumbenzoat (EuAB) | 0,2 g |

**Feste Zusammensetzung E (mit 5% Syloid®)**

| | |
|---|---|
| (6R)-5,6,7,8-Tetrahydro-L-biopterin·2HCl | 10 g |
| L-Cystein-HCl wasserfrei | 5 g |
| Syloid®AL 1 FP (5%) | 0,76 g |
| Natriumbenzoat (EuAB) | 0,2 g |

### Beispiel 4

### Stabilität in Abhängigkeit von der hinzugefügten Sulfhydrylverbindung

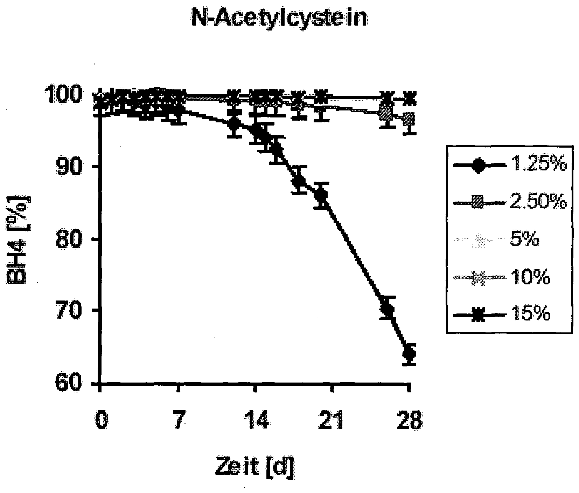

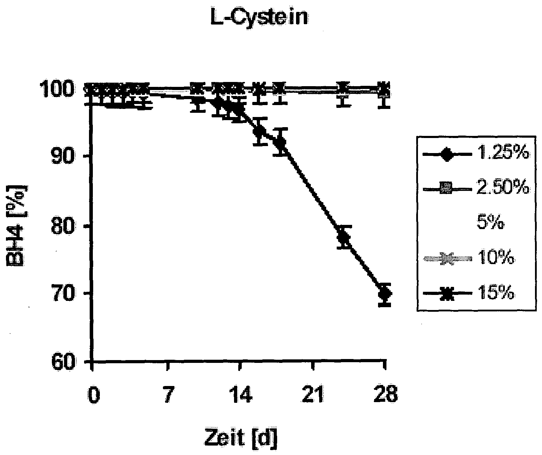

Der Einfluss von N-Acetylcystein (Fig. 1) und L-Cystein (Fig. 2) in verschiedenen Konzentrationen auf die Stabilität einer 10%igen (w/w) Tetrahydrobiopterin-Lösung wird bewertet. Flüssige Formulierungen werden bei Raumtemperatur gelagert, und Aliquote werden an vorbestimmten Zeitpunkten entnommen und hinsichtlich des Tetrahydro-biopterin-Gehalts analysiert, und zwar gemäß dem von Fukushima et al. beschriebenen differenzierten Oxidationsverfahren (Fukushima T., Nixon J.C. - Analysis of reduced forms of biopterin in biological tissues and fluids. - Anal Biochem., 102 (1), 176-188, 1980; Fukushima T., Nixon J.C. - Chromatographic analysis of pteridines. - Methods Enzymol., 66, 429-436, 1980). Die Ergebnisse dieser Untersuchung sind in den Figuren 1 und 2 dargestellt.

### Beispiel 5

### Langzeitstabilität von flüssigen BH₄-Zusammensetzungen

Die BH₄ gemäß der Formulierung A enthaltenden Pulversubstanzen werden in braune Glasflaschen von Valois übertragen, die mit bis zu 100 g Millipore-Ultrapure-Wasser gefüllt und mit den Aluscheiben und -kappen von Valois verschlossen werden. Die Hälfte der Flaschen wird zusätzlich in Alusäcken verpackt. Die Proben werden bei accellerierten Bedingungen (40°C ± 2°C und 75% ± 5% relative Feuchtigkeit) und Langzeitbedingungen (25°C ± 2°C bei 60% ± 5% relativer Feuchtigkeit) in den Klimaschrank gegeben und 6 Monate lang gelagert. Der BH4-Gehalt am Beginn des Versuchs und nach dem Zeitraum von 6 Monaten wird unter Anwendung des sauren und alkalischen Oxidationsverfahrens von Fukushima et al. bewertet. Tabelle 2 zeigt die relativen BH₄-Mengen, die nach einer Lagerung von 6 Monaten oder 12 Monaten gemessen werden, verglichen mit der Ausgangsmenge.

**Tabelle 2**

| **Bedingungen** | **25°C / 60% r.F.** | | **40°C / 75% r.F.** | |
|---|---|---|---|---|
| **Probe** | **1** | **2** | **3** | **4** |
| **Substanz** | **BH₄-Flüssigkeit - mit Alusack** | **BH₄-Flüssigkeit - ohne Alusack** | **BH₄-Flüssigkeit - mit Alusack** | **BH₄-Flüssigkeit - ohne Alusack** |
| **relative BH₄**- **Menge [%]: nach 6 Monaten** | 105,1 ± 0,2 | 103,1 ± 0,2 | 103,5 ± 0,1 | 100,2 ± 0,2 |
| **relative BH₄**- **Menge [%]: nach 12 Monaten** | 105,0 ± 0,2 | 102,9 ± 0,2 | n.d | n.d |

| | | | | |
|---|---|---|---|---|
| *n.d.: nicht durchgeführt* | | | | |

Es ist offensichtlich, dass der BH₄-Gehalt der flüssigen BH₄-Formulierung sich nicht einmal nach 6 Monaten unter accellerierten Lagerungsbedingungen verschlechtert.

## Patentansprüche

1. Orale pharmazeutische Darreichungsform, umfassend Tetrahydrobiopterin, oder einen metabolischen Vorläufer davon, **dadurch gekennzeichnet, dass** sie eine wässrige Lösung von Tetrahydrobiopterin oder eines metabolischen Vorläufers davon, und ein Antioxidans, in einem Abgabebehälter und eine Dosiereinrichtung zur dosierten Abgabe der wässrigen Lösung, umfasst, wobei das Antioxidans eine Sulfhydrylverbindung ist und wobei das Molverhältnis des Tetrahydrobiopterin oder metabolischen Vorläufers davon zur Sulfhydrylverbindung 1,5:1 bis 1:4 oder mehr, insbesondere von 1:1 bis 1:1,5 beträgt.

2. Pharmazeutische Darreichungsform nach Anspruch 1, **dadurch gekennzeichnet dass** die Sulfhydrylverbindung eine Cystein-Verbindung ist, insbesondere ausgewählt aus der Gruppe Cystein, und davon abgeleitete Verbindungen, insbesondere ausgewählt aus der Gruppe N-Acetylcystein, Homocystein, N-Acetylhomocystein, Cysteinmethylester, Cysteinethylester, Homocysteinmethylester oder Homocysteinethylester; oder Mischungen aus zwei oder mehreren verschiedenen Sulfhydrylverbindungen, gegebenenfalls in der Form eines Salzes, insbesondere in der Form eines Hydrochloridsalzes.

3. Pharmazeutische Darreichungsform nach einem der Ansprüche 1 oder 2, **dadurch gekennzeichnet, dass** das Tetrahydrobiopterin, oder der metabolische Vorläufer davon, in einer Konzentration von 0.5 Gew% bis 50 Gew%, insbesondere 2 Gew% bis 20 Gew%, insbesondere 5 Gew% bis 15 Gew%, vorliegt.

4. Pharmazeutische Darreichungsform nach einem der Ansprüche 1 bis 3, **dadurch gekennzeichnet, dass** der pH-Wert der wässrigen Lösung unterhalb von 5, insbesondere unterhalb von 2 liegt.

5. Pharmazeutische Darreichungsform nach einem der Ansprüche 1 bis 4, **dadurch gekennzeichnet, dass** die Dosiereinrichtung eine Abgabe von Aliquoten der wässrigen Lösung von 100 µl bis 2000 µl ermöglicht.

6. Pharmazeutische Darreichungsform gemäß einem der Ansprüche 1 bis 5, in der die Dosiereinrichtung mit dem Abgabebehälter fest verbunden ist.

7. Pharmazeutische Darreichungsform gemäß einem der Ansprüche 1 bis 6, **dadurch gekennzeichnet, dass** sie einen Kit umfasst, in der die wässrige Lösung in einem Abgabebehälter und die Dosiereinrichtung getrennt, aber zusammen in einer Packung verpackt sind.

8. Verwendung einer pharmazeutischen Darreichungsform gemäß einem der Ansprüche 1 bis 7 zur Herstellung eines Arzneimittels zur Behandlung von Phenylketonurie-Varianten, die durch einen Tetrahydrobiopterin-Mangel verursacht werden, insbesondere Hyperphenylalaninämie, durch Verabreichung von Tetrahydrobiopterin oder eines metabolischen Vorläufers davon an Kinder oder Säuglinge, insbesondere durch Verabreichung in Kombination mit Milch oder Babynahrung.

## Claims

1. An oral pharmaceutical dosage form, comprising tetrahydrobiopterin, or a metabolic precursor thereof, **characterized in that** it comprises an aqueous solution of tetrahydrobiopterin, or a metabolic precursor thereof, and an antioxidant in a dispensing container and a dosing means for the dosed dispensation of the aqueous solution, wherein the antioxidant is a sulfhydryl compound and wherein the molar ratio of the tetrahydrobiopterin, or the metabolic precursor thereof, to the sulfhydryl compound amounts to 1.5:1 to 1:4 or more, in particular to 1: 1 to 1:1.5.

2. A pharmaceutical dosage form according to claim 1, **characterized in that** the sulfhydryl compound is a cysteine compound, in particular selected from the group cysteine, and compounds derived therefrom, in particular selected from the group of N acetyl cysteine, homocysteine, N acetyl homocysteine, cysteine methyl ester, cysteine ethyl ester, homocysteine methyl ester or homocysteine ethyl ester; or mixtures of two or several different sulfhydryl compounds, optionally in the form of a salt, in particular in the form of a hydrochloride salt.

3. A pharmaceutical dosage form according to any of the claims 1 or 2, **characterized in that** the tetrahydrobiopterin, or the metabolic precursor thereof, is present in a concentration of 0.5 wt% to 50 wt%, in particular 2 wt% to 20 wt%, in particular 5 wt% to 15 wt%.

4. A pharmaceutical dosage form according to any of the claims 1 to 3, **characterized in that** the pH of the aqueous solution is below 5, in particular below 2.

5. A pharmaceutical dosage form according to any of the claims 1 to 4, **characterized in that** the dosing means provides for the dispensation of aliquots of the aqueous solution of 100 µl to 2000 µl.

6. A pharmaceutical dosage form according to any of the claims 1 to 5, wherein the dosing means is firmly connected with the dispensing container.

7. A pharmaceutical dosage form according to any of the claims 1 to 6, **characterized in that** it comprises a kit, in which the aqueous solution in a dispensing container and the dosing means are separated, but packaged together in one package.

8. The use of a pharmaceutical dosage form according to any of the claims 1 to 7 for the preparation of a medicament for treating phenylketonuria variants caused by a deficiency of tetrahydrobiopterin, in particular hyperphenylalaninemia, by means of administration of tetrahydrobiopterin, or a metabolic precursor thereof, to children or infants, in particular by administration in combination with milk or baby food.

## Revendications

1. Forme galénique pharmaceutique orale, comportant de la tétrahydrobioptérine, ou un précurseur métabolique de celle-ci, **caractérisée en ce qu'**elle comporte une solution aqueuse de tétrahydrobioptérine ou d'un précurseur métabolique de celle-ci, et un antioxydant, dans un récipient distributeur, et un dispositif de dosage pour la distribution dosée de la solution aqueuse, dans lequel l'antioxydant est un composé sulfhydryle et dans lequel le rapport molaire de la tétrahydrobioptérine ou du précurseur métabolique de celle-ci sur le composé sulfhydryle est supérieur ou égal à 1,5:1 à 1:4, en particulier va de 1:1 à 1:1,5.

2. Forme galénique pharmaceutique selon la revendication 1, **caractérisée en ce que** le composé sulfhydryle est un composé cystéine, en particulier choisi dans le groupe de la cystéine, et des composés découlant de celle-ci, en particulier sélectionnés dans le groupe de la n-acétylcystéine, de l'homocystéine, de la n-acétylhomocystéine, de l'ester de méthyle de cystéine, de l'ester d'éthyle de cystéine, de l'ester de méthyle d'homocystéine ou de l'ester d'éthyle d'homocystéine ; ou des mélanges de deux composés sulfhydryle différents ou plus, le cas échéant se présentant sous la forme d'un sel, en particulier sous la forme d'un sel de chlorhydrate.

3. Forme galénique pharmaceutique selon l'une quelconque des revendications 1 ou 2, **caractérisée en ce que** la tétrahydrobioptérine, ou le précurseur métabolique de celle-ci, est présente dans une concentration de 0,5 % en poids à 50 % en poids, en particulier de 2 % en poids à 20 % en poids, en particulier de 5 % en poids à 15 % en poids.

4. Forme galénique pharmaceutique selon l'une quelconque des revendications 1 à 3, **caractérisée en ce que** le pH de la solution aqueuse est inférieur à 5, en particulier inférieur à 2.

5. Forme galénique pharmaceutique selon l'une quelconque des revendications 1 à 4, **caractérisée en ce que** le dispositif de dosage permet une distribution d'aliquotes de la solution aqueuse de 100 µl à 2000 µl.

6. Forme galénique pharmaceutique selon l'une quelconque des revendications 1 à 5, dans laquelle le dispositif de dosage est relié fixement au récipient distributeur.

7. Forme galénique pharmaceutique selon l'une quelconque des revendications 1 à 6, **caractérisée en ce qu'**elle comporte un kit dans lequel la solution aqueuse dans un récipient distributeur et le dispositif de dosage sont séparés, mais emballés conjointement dans un emballage.

8. Utilisation d'une forme galénique pharmaceutique selon l'une quelconque des revendications 1 à 7 pour l'obtention d'un médicament permettant le traitement de variantes de phénylcétonurie, qui sont provoquées par un manque de tétrahydrobioptérine, en particulier l'hyperphénylalaninémie, par administration de tétrahydrobioptérine ou d'un précurseur métabolique de celle-ci à des enfants ou à des nourrissons, en particulier par une administration en combinaison avec du lait ou des aliments pour bébé.
